# EUROPÄISCHE PATENTSCHRIFT

(11) **EP 1 499 159 B1**
(45) Veröffentlichungstag und Bekanntmachung des Hinweises auf die Patenterteilung: **12.10.2011**
(21) Anmeldenummer: 04009707.3
(22) Anmeldetag: 23.04.2004
(51) Int. Cl.: A61F 11/14, G10K 11/178

(54) **Aktive Störgeräuschunterdrückung bei Hörhilfegeräten**
Active noise suppression in hearing-aids
Suppression active du bruit dans les prothèses auditives.

(30) Priorität: 16.07.2003 DE 10332119
(43) Veröffentlichungstag der Anmeldung: 19.01.2005
(73) Patentinhaber: Siemens Audiologische Technik GmbH, 91058 Erlangen (DE)
(72) Erfinder: Chalupper, Josef, 85307 Paunzhausen (DE); Lipski, Werner, 21680 Stade (DE); Rass, Uwe, 90480 Nürnberg (DE)
(74) Vertreter: Maier, Daniel Oliver

(56) Entgegenhaltungen:
- DE-A1- 3 719 963
- DE-A1- 3 908 673
- DE-A1- 4 010 372

## Beschreibung

Die Erfindung betrifft ein im Ohr tragbares Hörhilfegerät oder Hörhilfegerät mit im Ohr tragbarer Otoplastik, das wenigstens ein erstes Mikrofon zur Aufnahme eines ersten akustischen Signals und Abgabe eines ersten elektrischen Mikrofonsignals, eine Signalverarbeitungseinheit, einen ersten Hörer zur Abgabe eines ersten Hörersignals und einen Ventilationskanal umfasst, durch den die Belüftung des bei getragenem Hörhilfegerät bzw. bei getragener Otoplastik durch das Hörhilfegerät oder die Otoplastik eingeschlossenen Gehörgangvolumens erfolgt.

Bei einem im Ohr tragbaren Hörhilfegerät oder einem Hörhilfegerät mit im Ohr tragbarer Otoplastik wird durch das Gehäuse des Hörhilfegerätes bzw. die Otoplastik der äußere Gehörgang eines Ohres weitgehend schalldicht verschlossen. Üblicherweise ist das Gehäuse des im Ohr tragbaren Hörhilfegerätes bzw. die im Ohr tragbare Otoplastik von einem Ventilationskanal durchzogen, der zur Be- und Entlüftung des verschlossenen Abschnittes des äußeren Gehörganges dient. Die Querschnittsfläche dieses Ventilationskanals muss in der Regel verhältnismäßig klein gehalten werden, um akustische Störsignale, die unter Umgehung der elektrischen Signalverarbeitung durch das Hörhilfegerät den Ventilationskanal passieren, weitgehend zu vermeiden. Ein großer Durchmesser hat jedoch den Vorteil einer besseren Belüftung und eines geringeren Okklusionseffektes. Allerdings gelangt durch einen Ventilationskanal mit einem großen Querschnitt ein signifikanter tieffrequenter Direktschallanteil in das durch das Hörhilfegerät bzw. die Otoplastik eingeschlossene Gehörgangsvolumen. Je größer der Querschnitt des Ventilationskanals ist, desto höher liegt auch die Grenzfrequenz, unterhalb der ein merklicher Direktschall in den Ohrkanal einfällt.

Der Ventilationskanal stellt einen akustischen Bypass zu dem elektrischen Signalpfad durch das Hörhilfegerät dar. Störsignale, welche meist einen großen Anteil bei tiefen Frequenzen haben, können deshalb durch die elektrische Signalverarbeitung im Hörhilfegeräte nicht mehr gedämpft werden. Zur Vermeidung dieses Effekts wird der Querschnitt des Ventilationskanals so klein wie möglich ausgeführt. Dadurch kann jedoch häufig die ausreichende Be- und Entlüftung des eingeschlossenen Gehörgangvolumens nicht mehr gewährleistet werden.

Aus der DE 40 10 372 A1 ist ein Hörgerät mit einem in den äußeren Gehörgang eines Ohres einsetzbaren Ohrpassstück mit einem Ventilationskanal bekannt, bei dem ein akustisches Eingangssignal von einem ersten Mikrofon aufgenommen und in ein elektrisches Mikrofonsignal gewandelt, in einer Signalverarbeitungseinheit einer Signalverarbeitung unterzogen und durch einen ersten Hörer in ein akustisches Signal zurückgewandelt und in den Gehörgang des Hörgeräteträgers abgegeben wird. Bei dem bekannten Hörgerät befinden sich in dem Ventilationskanal ein zweites Mikrofon und ein zweiter Hörer, wobei von dem ersten Hörer erzeugte Schallwellen durch das zweite Mikrofon aufgenommen, phasengedreht und von dem zweiten Hörer wieder abgegeben werden, so dass aus dem eingeschlossenen Gehörgangvolumen durch den Ventilationskanal nach außen dringende Schallwellen des ersten Hörers weitgehend unterdrückt werden.

Aufgabe der vorliegenden Erfindung ist es, dem Gehör eines Hörgeräteträgers unter Umgehung der elektrischen Signalverarbeitung durch das Hörhilfegerät zugeführten Direktschall zu vermeiden.

Diese Aufgabe wird bei einem im Ohr tragbaren Hörhilfegerät oder Hörhilfegerät mit im Ohr tragbarer Otoplastik, das wenigstens ein erstes Mikrofon zur Aufnahme eines ersten akustischen Signals und Abgabe eines ersten elektrischen Mikrofonsignals, eine Signalverarbeitungseinheit, einen ersten Hörer zur Abgabe eines ersten Hörersignals und einen Ventilationskanal umfasst, durch den die Belüftung des bei getragenem Hörhilfegerät bzw. bei getragener Otoplastik durch das Hörhilfegerät oder die Otoplastik eingeschlossenen Gehörgangvolumens erfolgt, dadurch gelöst, dass ein zweites Mikrofon aus einem ersten Bereich des Ventilationskanals ein zweites akustisches Signal aufnimmt und in ein zweites elektrisches Mikrofonsignal wandelt, wobei das zweite elektrische Mikrofonsignal einer Filtereinrichtung zugeführt ist, wobei die Filtereinrichtung ein elektrisches Signal an einen zweiten Hörer abgibt und wobei der zweite Hörer in einen gegenüber dem ersten Bereich näher zum eingeschlossenen Gehörgangvolumen liegenden zweiten Bereich des Ventilationskanals ein zweites akustisches Hörersignal abgibt, welches ein von außen durch den Ventilationskanal in das eingeschlossene Gehörgangvolumen eindringendes akustisches Signal zumindest weitgehend unterdrückt.

Die Erfindung bietet den Vorteil, dass selbst bei einem verhältnismäßig großen Querschnitt des Ventilationskanals von außen durch den Ventilationskanal in den Gehörgang einfallender Direktschall weitgehend vermieden wird.

Bei einem Hörhilfegerät gemäß der Erfindung wird unter Berücksichtigung des Abstandes zwischen dem zweiten Mikrofon und dem zweiten Hörer sowie der Schallausbreitungsgeschwindigkeit in Luft ein akustisches Signal in dem Ventilationskanal erzeugt, welches im Bereich der Signalabgabe in den Ventilationskanal durch den zweiten Hörer gegenphasig zu dem von außen in den Ventilationskanal eindringenden akustischen Signal ist und dieses somit zumindest weitgehend auslöscht.

Da der durch den Ventilationskanal eindringende Direktschall insbesondere bei tiefen Frequenzen ein Problem darstellt, sieht eine Ausführungsform der Erfindung vor, den durch den Ventilationskanal eindringenden Direktschall lediglich im Bereich tiefer Frequenzen auszulöschen. Dies kann z.B. dadurch erfolgen, dass durch den zweiten Hörer lediglich ein tieffrequentes akustisches Hörersignal abgegeben wird.

Die Laufzeitverzögerung, Phasendrehung und ggf. Dämpfung des von dem zweiten Mikrofon aufgenommenen akustischen Signals erfolgt in einer Filtereinrichtung des Hörhilfegerätes. Diese kann in digitaler Schaltungstechnik ausgeführt sein. Alternativ kann die Phasendrehung auch mittels einer in Analogtechnik realisierten Schaltung erfolgen. Insbesondere können bei analoger Schaltungstechnik die notwendigerweise kurzen Durchlaufzeiten von dem zweiten Mikrofon zum zweiten Hörer besonders einfach realisiert werden. Zusätzliche A/D- bzw. D/A-Umsetzer erübrigen sich.

Eine Weiterbildung der Erfindung sieht ein drittes Mikrofon vor, das aus einem gegenüber dem zweiten Bereich näher zum eingeschlossenen Gehörgangvolumen liegenden dritten Bereich des Ventilationskanals ein drittes akustisches Signal aufnimmt und in ein drittes elektrisches Mikrofonsignal wandelt, wobei auch das dritte elektrische Mikrofonsignal der Filtereinrichtung zugeführt ist. In der Filtereinrichtung wird ein elektrisches Signal zur Ansteuerung des zweiten Hörers erzeugt, der dieses Signal in ein akustisches Signal wandelt und in den Ventilationskanal abgibt, so dass ein aus dem eingeschlossenen Gehörgangvolumen nach außen dringendes akustisches Signal zumindest weitgehend unterdrückt wird. Diese Weiterbildung bietet den Vorteil, dass neben dem akustischen Bypass durch den Ventilationskanal auch Rückkopplungen unterdrückt werden, die dadurch entstehen, dass ein akustisches Signal von dem ersten Hörer in den Gehörgang abgegeben wird und durch den Ventilationskanal erneut zum ersten Mikrofon gelangt und von diesem als akustisches Eingangssignal erfasst wird.

Eine alternative Ausführungsform zu der zuletzt genannten Weiterbildung sieht anstatt eines weiteren Mikrofons einen dritten Hörer vor, der ein drittes Hörersignal in einen gegenüber dem zweiten Bereich, in dem die Schallaufnahme durch das zweite Mikrofon erfolgt, weiter entfernt gegenüber dem eingeschlossenen Gehörgangvolumen liegenden dritten Bereich des Ventilationskanals ein drittes akustisches Hörersignal abgibt, welches ebenfalls ein aus dem eingeschlossenen Gehörgangvolumen nach außen dringendes akustisches Signal zumindest weitgehend unterdrückt.

Den beiden zuletzt genannten Weiterbildungsmöglichkeiten ist gemeinsam, dass ein akustisches Signal durch den Ventilationskanal, das unterdrückt werden soll, in Ausbreitungsrichtung dieses Signals zunächst von einem Mikrofon aufgenommen und in einer Filtereinrichtung phasengedreht wird, bevor ein in Ausbreitungsrichtung des akustischen Signals hinter dem Mikrofon angeordneter Hörer das so gefilterte Signal in den Ventilationskanal abgibt.

Eine Ausführungsform der Erfindung sieht vor, dass das zweite Mikrofon und der zweite Hörer zumindest teilweise in dem Ventilationskanal angeordnet sind. Dabei ist es auch möglich, dass sich der Querschnitt des Ventilationskanals im Bereich des Mikrofons bzw. des Hörers vergrößert. Ferner kann das zweite Mikrofon bzw. der zweite Hörer direkt an der Wand des Ventilationskanals angeordnet sein, so dass lediglich deren Schalleintritts- bzw. Schallaustrittsöffnung in den Ventilationskanal ragt.

Eine weitere Alternative sieht Schallkanäle zwischen dem Mikrofon bzw. dem Hörer und dem Ventilationskanal vor. Diese Ausführungsform erleichtert zwar die Anordnung des Mikrofons bzw. des Hörers in dem Hörhilfegerät bzw. in der Otoplastik, allerdings müssen dann auch die Laufzeiten der akustischen Signale durch diese Schallkanäle bei der Filterung berücksichtigt werden.

Insgesamt kann durch die Erfindung der Direktschallanteil im Ohrkanal bei gleichem Querschnitt des Ventilationskanals erheblich reduziert werden oder es ist ein größerer Querschnitt des Ventilationskanals möglich, ohne dass hierdurch ein Verlust der Wirkung einer Störgeräuschunterdrückung bei tiefen Frequenzen durch die elektrische Signalverarbeitung durch das Hörhilfegerät auftritt.

Die Erfindung wird nachfolgend anhand von Ausführungsbeispielen näher erläutert. Es zeigen:
Figur 1 ein in dem Ohr tragbares Hörhilfegerät mit einem Ventilationskanal, in dem ein zweites Mikrofon und ein zweiter Hörer angeordnet sind,
Figur 2 ein in dem Ohr tragbares Hörhilfegerät mit zwei Mikrofonen und einem Hörer in dem Ventilationskanal und
Figur 3 ein Hörhilfegerät mit einem Mikrofon und zwei Hörern in dem Ventilationskanal.

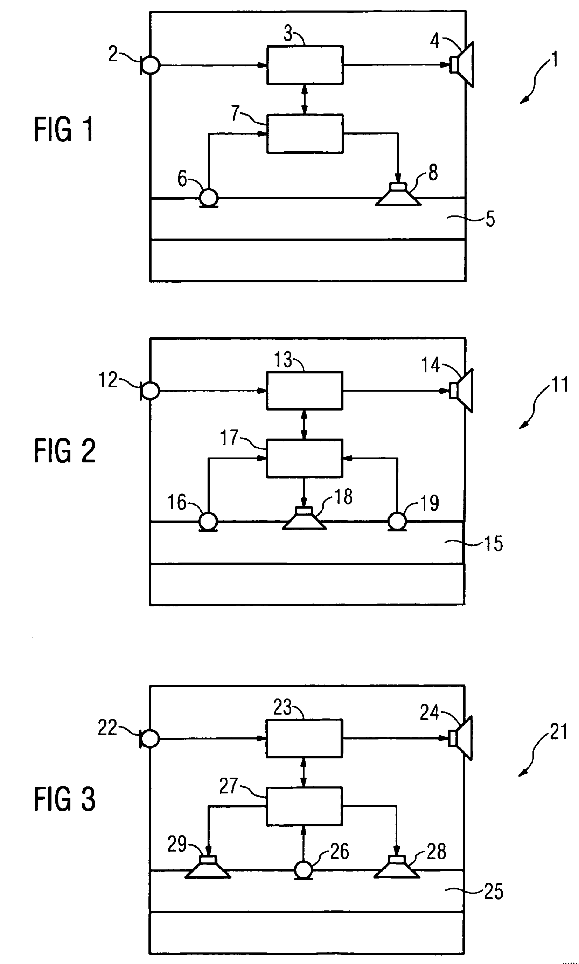

Figur 1 zeigt ein vereinfachtes Blockschaltbild für ein Hörhilfegerät 1 mit einem Mikrofon 2 zur Aufnahme eines akustischen Eingangssignals und Wandlung in ein elektrisches Mikrofonsignal. Das elektrische Mikrofonsignal ist einer Signalverarbeitungseinheit 3 zugeführt, in der die Signalverarbeitung und frequenzabhängige Verstärkung zum Ausgleich des individuellen Hörverlustes eines Hörgeräteträgers erfolgt. Das verarbeitete elektrische Signal ist schließlich einem Hörer 4 zugeführt, der das elektrische Signal in ein akustisches Signal wandelt und in einen Gehörgang des Hörgeräteträgers abgibt.

Zur Belüftung des von dem Hörhilfegerät 1 eingeschlossenen Gehörgangvolumens ist ein Ventilationskanal 5 vorhanden. Das Hörhilfegerät 1 gemäß der Erfindung weist ferner ein zweites Mikrofon 6 auf, welches in einem ersten Bereich des Ventilationskanals 5 ein akustisches Signal aufnimmt, in ein elektrisches Signal wandelt und einer Filtereinrichtung 7 zuführt. Aus der Filtereinrichtung 7 geht ein elektrisches Signal hervor, das über einen zweiten Hörer 8 in ein akustisches Signal gewandelt und in den Ventilationskanal 5 abgegeben wird. Gemäß der Erfindung sind das zweite Mikrofon 6 und der zweite Hörer 8 derart nebeneinander in dem Ventilationskanal 5 angeordnet, dass das zweite Mikrofon 6 bei getragenem Hörhilfegerät 1 näher an der dem Kopf des Hörgeräteträgers abgewandten Seite des Ventilationskanals 5 und der zweite Hörer 8 näher zum gegenüberliegenden, dem Kopf des Hörgeräteträgers zugewandten Ende des Ventilationskanals 5 liegt. Von außen durch den Ventilationskanal in den Gehörgang dringender Direktschall wird daher zunächst von dem zweiten Mikrofon 6 erfasst und unter Berücksichtigung der Laufzeit des akustischen Signals zwischen dem zweiten Mikrofon 6 und dem zweiten Hörer 8 durch die Filtereinrichtung 7 derart phasenverschoben, dass durch Abgabe dieses phasenverschobenen Signals mittels des zweiten Hörers 8 eine Auslöschung des Direktschalls in dem Ventilationskanal 5 erfolgt.

Die Erfindung bietet den Vorteil, dass der akustische Bypass, den der Ventilationskanal 5 gegenüber dem elektrischen Signalpfad durch das Hörhilfegerät 1, ausgehend von dem Mikrofon 2 über die Signalverarbeitungseinheit 3 und den Hörer 4, darstellt, beseitigt wird. Dadurch ist es möglich, dass der Querschnitt des Ventilationskanals 5 bis hin zu einer offenen Versorgung vergrößert werden kann.

Bei einer bevorzugten Ausführungsform der Erfindung besteht eine elektrische Verbindung zur Signalübertragung zwischen der Signalverarbeitungseinheit 3 und der Filtereinrichtung 7, so dass die aktive Störschallunterdrückung gemäß der Erfindung beispielsweise nur dann aktiv ist, wenn bei der Signalverarbeitungseinheit 3 ein bestimmtes Hörprogramm eingestellt ist. Weiterhin kann die aktive Störschallunterdrückung gemäß der Erfindung auch in Abhängigkeit von der augenblicklichen akustischen Umgebungssituation erfolgen, z.B. derart, dass diese nur dann durchgeführt wird, wenn das von dem Mikrofon 2 aufgenommene akustische Eingangssignal einen bestimmten Signalpegel übersteigt.

Eine Weiterbildung der Erfindung ist in Figur 2 dargestellt. Auch dabei erfolgt bei einem Hörhilfegerät 11 die Aufnahme eines akustischen Eingangssignals über ein erstes Mikrofon 12, eine Signalverarbeitung durch eine Signalverarbeitungseinheit 13 und schließlich die Abgabe eines akustischen Ausgangssignals in den Gehörgang eines Hörhilfegeräteträgers über einen ersten Hörer 14. Auch das Hörhilfegerät 11 weist einen Ventilationskanal 15 auf, aus dem mittels eines zweiten Mikrofons 16 ein akustisches Signal aufgenommen, in ein elektrisches Signal gewandelt und einer Filtereinrichtung 17 zugeführt wird. Das in der Filtereinrichtung 17 phasengedrehte Signal wird über einen zweiten Hörer 18 in den Ventilationskanal 15 abgegeben, wobei auch hierbei der zweite Hörer 18 gegenüber dem zweiten Mikrofon 16 näher bei dem dem Kopf zugewandten Ende des Ventilationskanals 15 angeordnet ist. Im Unterschied zum vorhergehenden Ausführungsbeispiel umfasst das Hörhilfegerät 11 ferner ein drittes Mikrofon 19, welches ein drittes akustisches Signal aufnimmt und in ein drittes elektrisches Mikrofonsignal wandelt, das ebenso wie das zweite elektrische Mikrofonsignal des zweiten Mikrofons 16 der Filtereinrichtung 17 zugeführt ist. Auch für das Mikrofon-Hörer-Paar 19, 18 wird durch die Filtereinrichtung 17 eine Phasendrehung durchgeführt, so dass ein von dem ersten Hörer 14 ausgehendes und durch den Ventilationskanal 15 nach außen laufendes akustisches Signal im Bereich des zweiten Hörers 18 ausgelöscht wird.

Diese Weiterentwicklung bietet den Vorteil, dass eine völlige Auslöschung aller den Ventilationskanal 15 passierenden akustischen Signale erfolgen kann. Zusätzlich zu den bereits genannten Vorteilen werden damit bei dem Hörhilfegerät 11 auch Rückkopplungen weitgehend vermieden.

Figur 3 zeigt eine Alternative zu dem Ausführungsbeispiel gemäß Figur 2. Auch bei diesem Ausführungsbeispiel wird bei einem Hörhilfegerät 21 von einem Mikrofon 22 ein akustisches Eingangssignal aufgenommen, in ein elektrisches Mikrofonsignal gewandelt und einer Signalverarbeitungseinheit 23 zugeführt. Das verarbeitete Signal wird von einem Hörer 24 in ein akustisches Signal gewandelt und in den Gehörgang des Hörgeräteträgers abgegeben. Auch bei dem Ausführungsbeispiel gemäß Figur 3 passiert ein von außen durch einen Ventilationskanal 25 in den Gehörgang gelangendes akustisches Signal zunächst ein zweites Mikrofon 26 und anschließend einen zweiten Hörer 28. In einer Filtereinrichtung 27 wird das von dem zweiten Mikrofon 26 aufgenommene und in ein elektrisches Mikrofonsignal gewandelte akustische Signal phasenverzögert, so dass im Bereich der Schallabgabe durch den zweiten Hörer 28 zumindest im Wesentlichen eine Auslöschung des von außen in den Gehörgang eindringenden Schallsignals erfolgt. Weiterhin wird von dem zweiten Mikrofon 26 auch ein akustisches Signal erfasst, das von dem ersten Hörer 24 in das eingeschlossene Gehörgangvolumen abgegeben und durch den Ventilationskanal 25 nach außen geleitet wird. Auch bei dem daraus hervorgehenden zweiten Mikrofonsignal erfolgt durch die Filtereinrichtung 27 eine Phasenverschiebung, so dass im Bereich eines dritten Hörers 28 eine Auslöschung des durch den Ventilationskanal 25 nach außen geleiteten akustischen Signals erfolgt. Auch diese Ausführungsform bietet den Vorteil, dass mit einer sehr kurzen Reaktionszeit sowohl ein von außen nach innen als auch ein von innen nach außen durch den Ventilationskanal 25 laufendes akustisches Signal unterdrückt werden kann.

Zusammenfassend soll bei einem im Ohr tragbaren Hörhilfegerät oder einem Hörhilfegerät mit im Ohr tragbarer Otoplastik das Eindringen von Direktschall durch einen Ventilationskanal 5, 15, 25 des Hörhilfegerätes bzw. der Otoplastik verhindert werden. Die Erfindung schlägt hierzu vor, mittels eines zweiten Mikrofons 6, 16, 26 in einem ersten Bereich des Ventilationskanals 5, 15, 25 ein akustisches Signal aus dem Ventilationskanal 5, 15, 25 aufzunehmen und in einer Filtereinrichtung 7, 17, 27 bezüglich der Phase derart zu verschieben, dass nach Abgabe des phasenverschobenen Signals in den Ventilationskanal 5, 15, 25 mittels eines zweiten Hörers 8, 18, 28 der Direktschall zumindest weitgehend ausgelöscht wird. Die Erfindung bietet den Vorteil, dass dadurch eine Vergrößerung des Querschnitts des Ventilationskanals 5, 15, 25, ja sogar eine offene Versorgung ermöglicht wird, ohne dass störender Direktschall in den Gehörgang des Hörgeräteträgers gelangt.

## Patentansprüche

1. Im Ohr tragbares Hörhilfegerät (1, 11, 21) oder Hörhilfegerät mit im Ohr tragbarer Otoplastik, das wenigstens ein erstes Mikrofon (2, 12, 22) zur Aufnahme eines ersten akustischen Signals und Abgabe eines ersten elektrischen Mikrofonsignals, eine Signalverarbeitungseinheit (3, 13, 23), einen ersten Hörer (4, 14, 24) zur Abgabe eines ersten Hörersignals und einen Ventilationskanal (5, 15, 25) umfasst, durch den die Belüftung des bei getragenem Hörhilfegerät (1, 11, 21) bzw. bei getragener Otoplastik durch das Hörhilfegerät oder die Otoplastik eingeschlossenen Gehörgangvolumens erfolgt, **dadurch gekennzeichnet, dass** ein zweites Mikrofon (6, 16, 26) aus einem ersten Bereich des Ventilationskanals ein zweites akustisches Signal aufnimmt und in ein zweites elektrisches Mikrofonsignal wandelt, wobei das zweite elektrische Mikrofonsignal einer Filtereinrichtung (7, 17, 27) zugeführt ist, wobei die Filtereinrichtung (7, 17, 27) ein elektrisches Signal an einen zweiten Hörer (8, 18, 28) abgibt und wobei der zweite Hörer (8, 18, 28) in einen gegenüber dem ersten Bereich näher zum eingeschlossenen Gehörgangvolumen liegenden zweiten Bereich des Ventilationskanals (5, 15, 25) ein zweites akustisches Hörersignal abgibt, welches ein von außen durch den Ventilationskanal (5, 15, 25) in das eingeschlossene Gehörgangvolumen eindringendes akustisches Signal zumindest weitgehend unterdrückt.

2. Im Ohr tragbares Hörhilfegerät (1, 11, 21) oder Hörhilfegerät mit im Ohr tragbarer Otoplastik nach Anspruch 1, **gekennzeichnet durch** ein drittes Mikrofon (19), das aus einem gegenüber dem zweiten Bereich näher zum eingeschlossenen Gehörgangvolumen liegenden Bereich des Ventilationskanals (15) ein drittes akustisches Signal aufnimmt und in ein drittes elektrisches Mikrofonsignal wandelt, wobei das dritte elektrische Mikrofonsignal der Filtereinrichtung (17) zugeführt ist und wobei die Filtereinrichtung (17) ein elektrisches Signal an den zweiten Hörer (18) abgibt, welches ein aus dem eingeschlossene Gehörgangvolumen nach außen dringendes akustisches Signal zumindest weitgehend unterdrückt.

3. Im Ohr tragbares Hörhilfegerät (1, 11, 21) oder Hörhilfegerät mit im Ohr tragbarer Otoplastik nach Anspruch 1, **gekennzeichnet durch** einen dritten Hörer (29), der ein drittes Hörersignal in einen gegenüber dem zweiten Bereich weiter entfernt zum eingeschlossenen Gehörgangvolumen liegenden Bereich des Ventilationskanals (25) ein drittes akustisches Hörersignal abgibt, welches ein aus dem eingeschlossene Gehörgangvolumen nach außen dringendes akustisches Signal zumindest weitgehend unterdrückt.

4. Im Ohr tragbares Hörhilfegerät (1, 11, 21) oder Hörhilfegerät mit im Ohr tragbarer Otoplastik nach einem der Ansprüche 1 bis 3, **dadurch gekennzeichnet, dass** das zweite Mikrofon (6, 16, 26) zumindest teilweise in dem Ventilationskanal (5, 15, 25) angeordnet ist.

5. Im Ohr tragbares Hörhilfegerät (1, 11, 21) oder Hörhilfegerät mit im Ohr tragbarer Otoplastik nach einem der Ansprüche 1 bis 4, **dadurch gekennzeichnet, dass** der zweite Hörer (8, 18, 28) zumindest teilweise in dem Ventilationskanal (5, 15, 25) angeordnet ist.

6. Im Ohr tragbares Hörhilfegerät (1, 11, 21) oder Hörhilfegerät mit im Ohr tragbarer Otoplastik nach einem der Ansprüche 1 bis 5, **dadurch gekennzeichnet, dass** das dritte Mikrofon (19) zumindest teilweise in dem Ventilationskanal (5, 15, 25) angeordnet ist.

7. Im Ohr tragbares Hörhilfegerät (1, 11, 21) oder Hörhilfegerät mit im Ohr tragbarer Otoplastik nach einem der Ansprüche 1 bis 6, **dadurch gekennzeichnet, dass** der dritte Hörer (29) zumindest teilweise in dem Ventilationskanal (5, 15, 25) angeordnet ist.

8. Im Ohr tragbares Hörhilfegerät (1, 11, 21) oder Hörhilfegerät mit im Ohr tragbarer Otoplastik nach einem der Ansprüche 1 bis 3, **dadurch gekennzeichnet, dass** das zweite Mikrofon (6, 16, 26) über einen Schallkanal mit dem Ventilationskanal verbunden ist.

9. Im Ohr tragbares Hörhilfegerät (1, 11, 21) oder Hörhilfegerät mit im Ohr tragbarer Otoplastik nach einem der Ansprüche 1 bis 3 oder nach Anspruch 8, **dadurch gekennzeichnet, dass** der zweite Hörer (8, 18, 28) über einen Schallkanal mit dem Ventilationskanal verbunden ist.

10. Im Ohr tragbares Hörhilfegerät (1, 11, 21) oder Hörhilfegerät mit im Ohr tragbarer Otoplastik nach einem der Ansprüche 1 bis 3 oder nach Anspruch 8 oder 9, **dadurch gekennzeichnet, dass** das dritte Mikrofon (19) über einen Schallkanal mit dem Ventilationskanal verbunden ist.

11. Im Ohr tragbares Hörhilfegerät (1, 11, 21) oder Hörhilfegerät mit im Ohr tragbarer Otoplastik nach einem der Ansprüche 1 bis 3 oder nach einem der Ansprüche 8 bis 10, **dadurch gekennzeichnet, dass** der dritte Hörer (29) über einen Schallkanal mit dem Ventilationskanal verbunden ist.

12. Im Ohr tragbares Hörhilfegerät (1, 11, 21) oder Hörhilfegerät mit im Ohr tragbarer Otoplastik nach einem der Ansprüche 1 bis 11, **dadurch gekennzeichnet, dass** bei dem Hörhilfegerät (1, 11, 21) mehrere Hörprogramme zur Anpassung der Signalverarbeitung im Hörhilfegerät (1, 11, 21) an unterschiedliche Umgebungssituationen einstellbar sind und die Unterdrückung eines den Ventilationskanal durchlaufenden akustischen Signals in Abhängigkeit des eingestellten Hörprogramms erfolgt.

## Claims

1. Hearing aid device (1, 11, 21) which can be worn in the ear or a hearing aid device with otoplasty which can be worn in the ear, which comprises at least a first microphone (2, 12, 22) for picking up a first acoustic signal and emitting a first electrical microphone signal, a signal processing unit (3, 13, 23), a first earphone (4, 14, 24) for emitting a first earphone signal and a ventilation channel (5, 15, 25), which is used for ventilating the volume of the auditory canal that is enclosed by the hearing aid device (1, 11, 21) when it is being worn or by the otoplasty when it is being worn, **characterized in that** a second microphone (6, 16, 26) picks up a second acoustic signal from a first region of the ventilation channel and converts it into a second electrical microphone signal, the second electrical microphone signal being fed to a filter device (7, 17, 27), the filter device (7, 17, 27) emitting an electrical signal to a second earphone (8, 18, 28) and the second earphone (8, 18, 28) emitting into a second region of the ventilation channel (5, 15, 25), which lies closer to the enclosed volume of the auditory canal than the first region, a second acoustic earphone signal, which at least largely suppresses an acoustic signal penetrating through the ventilation channel (5, 15, 25) from the outside into the enclosed volume of the auditory canal.

2. Hearing aid device (1, 11, 21) which can be worn in the ear or the hearing aid device with otoplasty which can be worn in the ear according to Claim 1, **characterized by** a third microphone (19), which picks up a third acoustic signal from a region of the ventilation channel (15) that lies closer to the enclosed volume of the auditory canal than the second region and converts it into a third electrical microphone signal, the third electrical microphone signal being fed to the filter device (17) and the filter device (17) emits an electrical signal to the second earphone (18), which at least largely suppresses an acoustic signal penetrating from the enclosed volume of the auditory canal to the outside.

3. Hearing aid device (1, 11, 21) which can be worn in the ear or the hearing aid device with otoplasty which can be worn in the ear according to Claim 1, **characterized by** a third earphone (29), which emits a third acoustic earphone signal into a region of the ventilation channel (25) that lies further away from the enclosed volume of the auditory canal than the second region, which third earphone signal at least largely suppresses an acoustic signal penetrating from the enclosed volume of the auditory canal to the outside.

4. Hearing aid device (1, 11, 21) which can be worn in the ear or the hearing aid device with otoplasty which can be worn in the ear according to one of Claims 1 to 3, **characterized in that** the second microphone (6, 16, 26) is arranged at least partly in the ventilation channel (5, 15, 25).

5. Hearing aid device (1, 11, 21) which can be worn in the ear or the hearing aid device with otoplasty which can be worn in the ear according to one of Claims 1 to 4, **characterized in that** the second earphone (8, 18, 28) is arranged at least partly in the ventilation channel (5, 15, 25).

6. Hearing aid device (1, 11, 21) which can be worn in the ear or the hearing aid device with otoplasty which can be worn in the ear according to one of Claims 1 to 5, **characterized in that** the third microphone (19) is arranged at least partly in the ventilation channel (5, 15, 25).

7. Hearing aid device (1, 11, 21) which can be worn in the ear or the hearing aid device with otoplasty which can be worn in the ear according to one of Claims 1 to 6, **characterized in that** the third earphone (29) is arranged at least partly in the ventilation channel (5, 15, 25).

8. Hearing aid device (1, 11, 21) which can be worn in the ear or the hearing aid device with otoplasty which can be worn in the ear according to one of Claims 1 to 3, **characterized in that** the second microphone (6, 16, 26) is connected to the ventilation channel via a sound channel.

9. Hearing aid device (1, 11, 21) which can be worn in the ear or the hearing aid device with otoplasty which can be worn in the ear according to one of Claims 1 to 3 or according to Claim 8, **characterized in that** the second earphone (8, 18, 28) is connected to the ventilation channel via a sound channel.

10. Hearing aid device (1, 11, 21) which can be worn in the ear or the hearing aid device with otoplasty which can be worn in the ear according to one of Claims 1 to 3 or according to Claim 8 or 9, **characterized in that** the third microphone (19) is connected to the ventilation channel via a sound channel.

11. Hearing aid device (1, 11, 21) which can be worn in the ear or the hearing aid device with otoplasty which can be worn in the ear according to one of Claims 1 to 3 or according to one of Claims 8 to 10, **characterized in that** the third earphone (29) is connected to the ventilation channel via a sound channel.

12. Hearing aid device (1, 11, 21) which can be worn in the ear or the hearing aid device with otoplasty which can be worn in the ear according to one of Claims 1 to 11, **characterized in that** a number of hearing programs for adapting the signal processing in the hearing aid device (1, 11, 21) to different ambient situations can be set on the hearing aid device (1, 11, 21) and the suppression of an acoustic signal passing through the ventilation channel takes place in dependence on the hearing program that is set.

## Revendications

1. Prothèse ( 1, 11, 21 ) auditive pouvant se porter dans l'oreille ou prothèse auditive ayant un plastique auriculaire pouvant se porter dans l'oreille, qui comprend au moins un premier microphone ( 2, 12, 22 ) pour la réception d'un premier signal acoustique et l'émission d'un premier signal électrique de microphone, une unité ( 3, 13, 23 ) de traitement du signal, un premier écouteur ( 4, 14, 24 ) pour l'émission d'un premier signal d'écouteur et un canal ( 5, 15, 25 ) de ventilation, par lequel s'effectue l'aération du volume du tuyau auditif enfermé, lorsque la prothèse ( 1, 11, 21 ) auditive est portée ou lorsque le plastique auriculaire est porté, par la prothèse auditive ou par le plastique auriculaire, **caractérisée en ce qu'**un deuxième microphone ( 6, 16, 26 ) reçoit un deuxième signal acoustique d'une première zone du canal de ventilation et le transforme en un deuxième signal électrique de microphone, le deuxième signal électrique de microphone étant envoyé à un dispositif ( 7, 17, 27 ) de filtrage, le dispositif ( 7, 17, 27 ) de filtrage émettant un signal électrique vers un deuxième écouteur ( 8, 18, 28 ) et le deuxième écouteur ( 8, 18, 28 ) émettant un deuxième signal acoustique d'écouteur dans une deuxième zone du canal ( 5, 15, 25 ) de ventilation, plus près du volume du tuyau auditif enfermé que la première zone, lequel deuxième signal acoustique d'écouteur supprime, au moins dans une grande mesure, un signal acoustique pénétrant de l'extérieur par le canal ( 5, 15, 25 ) de ventilation dans le volume du tuyau auditif enfermé.

2. Prothèse ( 1, 11, 21 ) auditive pouvant se porter dans l'oreille ou prothèse auditive ayant un plastique auriculaire pouvant se porter dans l'oreille suivant la revendication 1, **caractérisée par** un troisième microphone ( 19 ) qui reçoit un troisième signal acoustique d'une zone du canal ( 15 ) de ventilation plus proche du volume du tuyau auditif enfermé que la deuxième zone et le transforme en un troisième signal électrique de microphone, le troisième signal électrique de microphone étant envoyé à un dispositif ( 17 ) de filtrage et le dispositif ( 17 ) de filtrage émettant un signal électrique vers le deuxième écouteur ( 18 ), lequel signal électrique supprime au moins dans une grande mesure un signal acoustique allant vers l'extérieur à partir du volume du tuyau auditif enfermé.

3. Prothèse ( 1, 11, 21 ) auditive pouvant se porter dans l'oreille ou prothèse auditive ayant un plastique auriculaire pouvant se porter dans l'oreille suivant la revendication 1, **caractérisée par** un troisième écouteur ( 29 ) qui émet un troisième signal d'écouteur dans une zone du canal ( 25 ) de ventilation plus éloigné du volume du tuyau auditif enfermé que la deuxième zone, troisième signal acoustique d'écouteur lequel signal acoustique supprime, au moins dans une grande mesure, un signal acoustique allant vers l'extérieur à partir du volume enfermé du tuyau auditif.

4. Prothèse ( 1, 11, 21 ) auditive pouvant se porter dans l'oreille ou prothèse auditive ayant un plastique auriculaire pouvant se porter dans l'oreille suivant l'une des revendications 1 à 3, **caractérisée en ce que** le deuxième microphone ( 6, 16, 26 ) est disposé au moins en partie dans le canal ( 5,15,25 ) de ventilation.

5. Prothèse ( 1, 11, 21 ) auditive pouvant se porter dans l'oreille ou prothèse auditive ayant un plastique auriculaire pouvant se porter dans l'oreille suivant l'une des revendications 1 à 4, **caractérisée en ce que** le deuxième écouteur ( 8, 18, 28 ) est disposé au moins en partie dans le canal ( 5, 15, 25 ) de ventilation.

6. Prothèse ( 1, 11, 21 ) auditive pouvant se porter dans l'oreille ou prothèse auditive ayant un plastique auriculaire pouvant se porter dans l'oreille suivant l'une des revendications 1 à 5, **caractérisée en ce que** le troisième microphone ( 19 ) est disposé au moins en partie dans le canal ( 5, 15, 25 ) de ventilation.

7. Prothèse ( 1, 11, 21 ) auditive pouvant se porter dans l'oreille ou prothèse auditive ayant un plastique auriculaire pouvant se porter dans l'oreille suivant l'une des revendications 1 à 6, **caractérisée en ce que** le troisième écouteur ( 29 ) est disposé au moins en partie dans le canal ( 5, 15, 25 ) de ventilation

8. Prothèse ( 1, 11, 21 ) auditive pouvant se porter dans l'oreille ou prothèse auditive ayant un plastique auriculaire pouvant se porter dans l'oreille suivant l'une des revendications 1 à 3, **caractérisée en ce que** le deuxième microphone ( 6, 16, 26 ) communique avec le canal de ventilation par un canal pour le son.

9. Prothèse ( 1, 11, 21 ) auditive pouvant se porter dans l'oreille ou prothèse auditive ayant un plastique auriculaire pouvant se porter dans l'oreille suivant l'une des revendications 1 à 3 ou suivant la revendication 8, **caractérisée en ce que** le deuxième écouteur ( 8, 18, 28 ) communique avec le canal de ventilation par un canal pour le son.

10. Prothèse ( 1, 11, 21 ) auditive pouvant se porter dans l'oreille ou prothèse auditive ayant un plastique auriculaire pouvant se porter dans l'oreille suivant l'une des revendications 1 à 3 ou suivant la revendication 8 ou 9, **caractérisée en ce que** le troisième microphone ( 19 ) communique avec le canal de ventilation par un canal pour le son.

11. Prothèse ( 1, 11, 21 ) auditive pouvant se porter dans l'oreille ou prothèse auditive ayant un plastique auriculaire pouvant se porter dans l'oreille suivant l'une des revendications 1 à 3 ou suivant l'une des revendications 8 à 10, **caractérisée en ce que** le troisième écouteur ( 29 ) communique avec le canal de ventilation par un canal pour le son.

12. Prothèse ( 1, 11, 21 ) auditive pouvant se porter dans l'oreille ou prothèse auditive ayant un plastique auriculaire pouvant se porter dans l'oreille suivant l'une des revendications 1 à 11, **caractérisée en ce que**, dans la prothèse ( 1, 11, 21 ) auditive, plusieurs programmes auditifs d'adaptation du traitement du signal dans la prothèse ( 1, 11, 21 ) auditif peuvent être établis en fonction de situations ambiantes différentes et la suppression d'un signal acoustique passant dans le canal de ventilation s'effectue en fonction du programme auditif établi.
